# EUROPÄISCHE PATENTSCHRIFT

(11) **EP 2 735 286 B1**
(45) Veröffentlichungstag und Bekanntmachung des Hinweises auf die Patenterteilung: **06.03.2019**
(21) Anmeldenummer: 13005487.7
(22) Anmeldetag: 25.11.2013
(51) Int. Cl.: A61F 2/44

(54) **Expandierbarer Cage zur interkorporellen Fusion von Lendenwirbeln**
Expandable cage for intracorporeal fusion of lumbar vertebrae
Cage expansible pour la fusion intercorporelle de vertèbres lombaires

(30) Priorität: 26.11.2012 DE 102012023042
(43) Veröffentlichungstag der Anmeldung: 28.05.2014
(73) Patentinhaber: spontech spine intelligence AG, 70182 Stuttgart (DE)
(72) Erfinder: Pabst, Martin, D-78166 Donaueschingen (DE); Taddia, Lino, D-78333 Stockach (DE); Pandorf, Thomas, D-73249 Wernau (DE); Spitzenberg, Marcus, D-72116 Mössingen (DE)
(74) Vertreter: Ostertag & Partner Patentanwälte mbB

(56) Entgegenhaltungen:
- EP-A1- 1 236 451
- EP-B1- 1 233 732
- US-A1- 2010 049 324
- US-A1- 2012 029 637
- US-B2- 6 723 128

## Beschreibung

### Hintergrund und Gebiet der Erfindung

Die vorliegende Erfindung betrifft einen Cage zur interkorporellen Fusion von Lendenwirbeln gemäß dem Oberbegriff des vorliegenden Patentanspruchs 1 .

Das technische Gebiet, auf das sich die vorliegende Erfindung bezieht, ist die Wirbelsäulenchirurgie. Zur Behandlung von Personen mit degenerativen Veränderungen der Lendenwirbelsäule, bei denen eine mono- oder multisegmentale Fusion angezeigt ist, aber auch bei Fällen, in denen eine Dekompression der neuralen Strukturen beabsichtigt wird, haben sich insbesondere zwei Operationstechniken bewährt, die einen Eingriff ohne transabdominalem zusätzlichen Zugang erlauben. Bei der sogenannten PLIF-Operationstechnik (Posterior Lumbar Intervertebral Fusion) werden über einen posterioren Zugang pro Bandscheibenfach vorzugsweise zwei sogenannte Cages implantiert. Die implantierten Cages dienen als Platzhalter im Zwischenwirbelspalt und stellen den vom Operateur gewünschten Abstand der angrenzenden Wirbelkörper wieder her. Zusätzlich werden die Wirbel durch ein posterior angebrachtes Stab-Schrauben-System fixiert also stabilisiert. Die zweite, TLIF (Transforaminal Lumbar Intervertebral Fusion) genannte Operationstechnik ist eine aus der PLIF-Operationstechnik weiterentwickelte Operationsmethode. Bei diesem Verfahren wird durch einen dorsolateralen Zugang vorzugsweise ein einzelner Cage implantiert. Auch bei dieser Technik wird eine posteriore Stabilisierung angebracht. Beide Operationstechniken haben zum Ziel, eine Fusion, also eine knöcherne Verbindung, der betroffenen Segmente zu erreichen. Diese kann durch die Zugabe von synthetischen oder biologischen Knochenersatzmaterial verbessert werden. Die Cages werden üblicherweise aus Metallen oder aus Kunststoffen gefertigt.

### Stand der Technik

Für beide Operationsmethoden sind aus dem Stand der Technik im Längsschnitt annähernd trapezoide oder keilförmige Cages mit einem rechteckigen Querschnitt bekannt, die die Krümmung der Wirbelsäule aufgrund ihrer Form korrigieren. Auch sind bereits sogenannte expandierbare Cages der genannten Art bekannt, die den Vorteil bieten, dass sie ein leichteres Einführen aufgrund niedrigerer Eintrittshöhe ermöglichen.

In der EP 1 290 985 B1 ist ein derartiger expandierbarer Cage offenbart, der ein Spreizelement zum Expandieren des Cages in seinem Inneren aufweist, wobei eine auf Gleitschienen mit schrägen Aufgleitflächen bewegliche Distanzscheibe einschiebbar ist, so dass in Abhängigkeit von der Tiefe des Einschubs des Distanzelementes eine unterschiedliche Aufspreizung des Cages bis zu einer durch einen Vorsprung oder eine Stufe ausgebildeten Endstellung erfolgt.

Zwar bietet dieser bekannte Cage aufgrund seiner Expandierbarkeit den oben genannten Vorteil, der bis zur Endstellung notwendige Einschiebevorgang der Distanzscheibe zur Spreizung des Cages ist aber aufwendig und erfordert vom Operateur viel Gefühl und Geschick. Bei einem nur halb expandierten Cage befindet sich das Distanzelement in der Mitte des Cages, die vorderen Enden kragen über und eine sichere Lastabtragung ist nicht gewährleistet. Zudem muss die Distanzscheibe zwangsgeführt werden, um ein Verkippen zu vermeiden. Eine klare Kontrolle über den erreichten Aufspreizungsgrad sowie die Kontrolle des sicheren Erreichens der Endstellung verbleibt ebenso schwierig.

Aus der DE 101 13 689 C1 ist ein gattungsgemäßer expandierbarer Cage bekannt, der die zuvor genannten Nachteile dadurch vermeidet, dass das Aufspreizen des Cages durch ein an seinem vorderen Ende gelagertes drehbares Spreizelement erfolgt. Hierzu weist dieser Cage zwei im Wesentlichen parallel zueinander verlaufende Arme auf, die an ihren Außenseiten Stützflächen für benachbarte Wirbelköper ausbilden, und an einem Ende über eine Brücke verbunden sind. An ihren jeweiligen anderen Enden der Arme befindet sich ein zwischenliegendes und in die Enden der Arme eingreifendes ellipsenförmiges Spreizelement, das um eine parallel zu den Armen verlaufende Rotationsachse verdrehbar ist. Dadurch können die Arme je nach Winkelstellung des Spreizelements mehr oder weniger aufgespreizt werden. Im nicht expandierten Zustand des Cages ruhen die Außenflächen des Spreizelementes mit dem geringeren Krümmungsradius in ovalen Ausnehmungen der Arme. Im expandierten Zustand liegen die Außenflächen des Spreizelementes mit dem größeren Krümmungsradius jeweils in an beiden Seiten durch Stufen geringer Höhe begrenzte Vertiefungen der Arme. Das Spreizelement lässt sich von außen in nutenförmige Vertiefungen beider Arme einsetzen, so dass es auf diese Weise gegen eine Verschiebung in axialer Richtung gesichert ist.

Obwohl damit eine leichtere und schnellere Einstellbarkeit des Cages in die expandierte Stellung erreicht wird, ergibt sich aufgrund der eingeschränkten Kontaktfläche zwischen dem Spreizelement und den Armen im expandierten Zustand nur eine geringe Fähigkeit zur Lastaufnahme, zumal unter Belastung ein Verdrehen des Spreizelementes um die Rotationsachse und ein Verkippen der Arme um parallel zu der Rotationsachse liegende Achsen nicht sicher verhindert werden kann.

Aus der US 6,723,128 B2 ist ein monolithisch aufgebauter expandierbarer Cage mit einem zwischen zwei Armen in einer Einführungsöffnung bündig eingebrachten drehbaren Spreizelement bekannt, dessen jeweilige, die Arme stützende Kontaktflächen verbreitert sind und mithin eine höhere Lastabtragung ermöglichen. Allerdings ist unter Belastung ein Verkippen der Arme um parallel zur Längsachse des Cages liegende Achsen immer noch möglich, da die quer zur Längsachse des Cages existierende Breite der Kontaktflächen verhältnismäßig klein gegenüber der Breite des Cages ist. Auch ist ein Verkippen des Spreizelementes selbst um eine senkrecht zur Längsachse des Cages stehenden Querachse nicht ausgeschlossen. Der Expansionsweg ist eingeschränkt.

Die EP 1 233 732 B1 (DE 601 19 279 T2) offenbart einen expandierbaren Cage mit zwei spreizbaren, wahlweise auch gelenkig miteinander verbundenen Armen mit einem dazwischenliegenden drehbaren Spreizelement. Das plattenförmige Spreizelement hat in bevorzugter Form eine modifizierte rechteckige oder rhomboide Form mit diagonal gegenüberliegenden abgerundeten Ecken, um die Drehung des Spreizelementes beim Überführen vom nicht expandierten in den expandierten Zustand des Cages zu erleichtern und eine substantielle Überdistraktion der benachbarten Wirbelkörper bei diesem Vorgang zu vermeiden. Das Spreizelement wird in Führungen der zwei expandierbaren Arme gehalten bzw. geführt. Zwar ist auch hier ein Verkippen des Spreizelementes um eine senkrecht zur Längsachse des Cages stehenden Querachse, insbesondere bei Belastung des Cages im expandierten Zustand, möglich, ein Verkippen der Arme um eine parallel zur Längsachse des Cages liegende Achse wird aber durch aufwendige, stabilisierende Teilwandstrukturen der Arme selbst vermieden. Diese erhöhen nicht nur die Steifigkeit der Arme sondern begrenzen zugleich auch das Vorhandensein möglichst großer seitlicher Aussparungen im Cage, die zur besseren radiologischen Durchdringung vorteilhaft sind.

### Aufgabe und Lösung

Der vorliegenden Erfindung liegt die Aufgabe zugrunde, einen expandierbaren Cage zur interkorporellen Fusion von Lendenwirbeln zu schaffen, der einfach und ohne großen Zeitaufwand in seinen expandierten Zustand bringbar ist und der zugleich eine hohe und sichere Lastabtragung gewährleistet.

Diese Aufgabe wird durch einen expandierbaren Cage zur interkorporellen Fusion von Lendenwirbeln nach dem Oberbegriff des vorliegenden Patentanspruchs 1 dadurch gelöst,
dass das Spreizelement aus einem drehbaren länglichen, insbesondere zylinderförmigen Grundkörper besteht, der ein oder zwei einstückig damit verbundene rechteckige Platten mit in Längsrichtung des Cages verlaufenden abgerundeten Kanten sowie zwei gegenüberliegende, radial ausgerichtete und in Längsrichtung des Cages verlaufende Rippen aufweist,
dass die rechteckigen Platten mit ihren Schmalseiten in korrespondierende Nuten, die in den Innenseiten der Arme quer verlaufen, sowohl im nicht expandierten als auch im expandierten Zustand des Cages eingreifen,
dass sowohl die Rippen als auch der Grundkörper jeweils paarweise gegenüberliegende gekrümmte Auflageflächen an ihrem jeweiligen äußeren Umfang aufweisen, die alternativ und abhängig vom Spreizzustand des Cages in korrespondierenden, in Längsrichtung des Cages verlaufenden und an den Innenseiten der Arme vorhandenen Rinnen zur drehbaren Lagerung aufliegen, und
dass zumindest im hinteren, zur brückenähnlichen Verbindung (15) hin liegenden Teil des Spreizelementes (3) eine Vorrichtung zum drehbaren Antrieb des Spreizelementes (3) vorhanden ist.

Neben der schnellen, eine einfache Drehung um 90° mittels eines geeigneten Instruments erfordernden Einstellbarkeit des Cages, um vom nicht expandierten in den expandierten Zustand und umgekehrt zu gelangen, ist ein besonderer Vorteil darin zu sehen, dass in beiden Zuständen jeweils relativ großflächige, in rechtem Winkel zueinander stehende längliche Kontaktflächen zwischen dem Spreizelement und den die Wirbelkörper abstützenden Armen vorhanden sind, die nicht nur eine hohe Lastabtragung sondern gleichzeitig auch ein Umkippen des Spreizelements unter Last verhindern. In Längsrichtung und in Querrichtung zum Cage gerichtete Verschiebungen des Spreizelements werden in beiden Zuständen ebenfalls verhindert. Weitere Vorteile ergeben sich durch Antriebsmöglichkeiten des Spreizelementes von posterior und anterior, wobei letztere zweckmäßig als Notantrieb für Revisions-Operationen zur Verfügung stehen kann. Zur besseren radiologischen Untersuchung weist der Cage offene Seitenwände auf.

Weitere zweckmäßige oder vorteilhafte Ausgestaltungen der Erfindung sind Gegenstand der Unteransprüche 2 bis 24.

So zeigen die Ansprüche 2 und 3 vorteilhafte Ausgestaltungen und zweckmäßige Kombinationen von Instrumentenkopplungen zum drehbaren Antrieb des Spreizelementes auf.

Die im Anspruch 4 definierte Form der Nuten ermöglicht beim Spreizvorgang eine annähern parallele Spreizung des Cages zur Frontalebene, im expandierten Zustand eine vergrößerte Auflagefläche.

Mit der in Anspruch 5 angegebenen Ausgestaltung der rechteckigen Platten ist eine vorteilhafte, zusätzliche Arretierung des Cages im expandierten Zustand gegeben.

Die in Anspruch 6 angegebenen zusätzlichen Ausgestaltungen der Platten erlauben eine von einem Einstellinstrument unabhängige, vorteilhafte Lagekontrolle des Spreizelementes im Cage durch ein bildgebendes Verfahren.

Durch die in Anspruch 7 angegeben Ausbildung wird ein Herausspringen des Spreizelementes in eine Fehlstellung verhindert.

Durch die im Anspruch 8 aufgezeigte Ausbildung wird eine sichere Anbindung mit einem Instrument erzielt und zugleich eine Öffnung zum Einbringen von künstlichem Knochenmaterial bereitgestellt.

Die monolithische Ausbildung des Hauptkörpers des Cage nach Anspruch 9 erlaubt eine vereinfachte Herstellung. Durch die hiermit gegebene Notwendigkeit des Vorhandenseins biegsamer spreizbarer Arme, die durch verformbare dünnere Armbereiche, z. B. mittels ovaler Ausformungen, erzielt werden kann, ergeben sich größtmögliche offene laterale Seiten, die eine bessere radiologische Durchdringung erlauben.

Mit der in den Ansprüchen 10 aufgeführten Ausgestaltung des Hauptkörpers mit einem oder zwei scharnierartigen Gelenken wird eine geringere Durchbiegung der Arme im Falle von differierenden Lasteinleitungssituationen erzielt.

Mit den im Anspruch 11 angegebenen Ausgestaltungen wird eine bessere Anpassung an die Form der Wirbelkörper erreicht, ein leichteres Einführen des Cage erzielt sowie eine Primärverankerung erhalten.

Mit der in den Ansprüchen 12 beanspruchten Grundform der Arme ergibt sich eine zweckmäßige Ausgestaltungen für die genannten verschiedenen Operationstechniken. Die Grundform kann aber auch parallelogrammförmig und insbesondere rechteckig sein oder eine elliptische Gestalt haben.

Wenn die Arme vertikale Aussparungen aufweisen, wird eine Möglichkeit zur Knochendurchdringung als sekundäre Verankerung geschaffen.

Die in den Armen befindlichen vertikalen Aussparungen können jeweils als ein im Wesentlichen teilelliptisch nach posterior kleiner werdendes Fenster oder als Langlöcher ausgestaltet sein, die in parallel zueinander verlaufenden Reihen mit zwei oder drei Langlöchern pro Reihe angeordnet sind. Alternativ hierzu können die in den Armen befindlichen vertikalen Aussparungen jeweils als ein im Wesentlichen rechteckiges Fenster ausgeführt sein oder aus hintereinander liegenden Bohrungen bestehen, die in Längsrichtung des Cages in mehreren, parallel zueinander verlaufenden Reihen matrixähnlich angeordnet sind. Ferner können die vertikalen Aussparungen jeweils aus 3 schmalen, gleich langen und in Längsrichtung des Cages parallel zueinander verlaufenden Durchbrechungen oder aus paarweisen seitlichen Ausbrüchen bestehen. Mit diesen an verschiedene Biegebelastungen der Arme angepasste Formen vertikaler Aussparungen lassen sich wahlweise maximale Knochendurchdringungen erzielen.

Die im Anspruch 13 angegebene Maßnahme gibt eine zweckmäßige Ausgestaltung eines Cages an.

### Ausführungsbeispiel

Im Folgenden wird eine bevorzugte Ausführungsform der Erfindung unter Bezugnahme auf die beiliegenden Figuren 1 - 12 näher erläutert.

Dabei zeigen:
Fig. 1: einen horizontalen medialen Längsschnitt durch den monolithischen Hauptkörper eines erfindungsgemäßen Cages mit auf dem unteren Arm, in expandierter Lage eingelegtem Spreizelement
Fig. 2: die frontseitige Ansicht des Spreizelementes nach Figur 1
Fig. 3: den erfindungsgemäßen Cage nach Figur 1 in teilweise gespreiztem Zustand
Fig. 4: den erfindungsgemäßen Cage nach Figur 3 im gespreizten Zustand
Fig. 5: einen horizontalen medialen Längsschnitt durch den erfindungsgemäßen Cage nach Figur 4 mit verbundenem Instrument
Fig. 6: einen vertikalen medialen Längsschnitt durch den erfindungsgemäßen Cage nach Figur 4 im nicht gespreizten Zustand
Fig. 7: einen vertikalen medialen Längsschnitt durch den erfindungsgemäßen Cage nach Figur 4 im gespreizten Zustand
Fig. 8 den erfindungsgemäßen Hauptkörper (2) des Cages (1) nach Figur 4 in seitlicher, nicht gespreizter Ansicht
Fig. 9 a - d verschiedene Grundformen erfindungsgemäßer Cages in Draufsicht
Fig. 10 a - f verschieden Formen vertikaler Aussparungen
Fig. 11 a - c, d - f Cages mit einem bzw. zwei scharnierartigen Gelenken
Fig. 12 a bis g verschiedene Ausführungen erfindungsgemäßer Spreizelemente

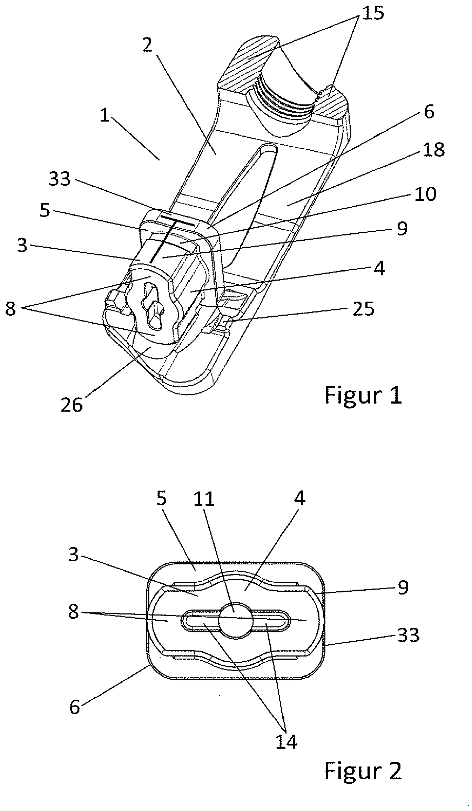

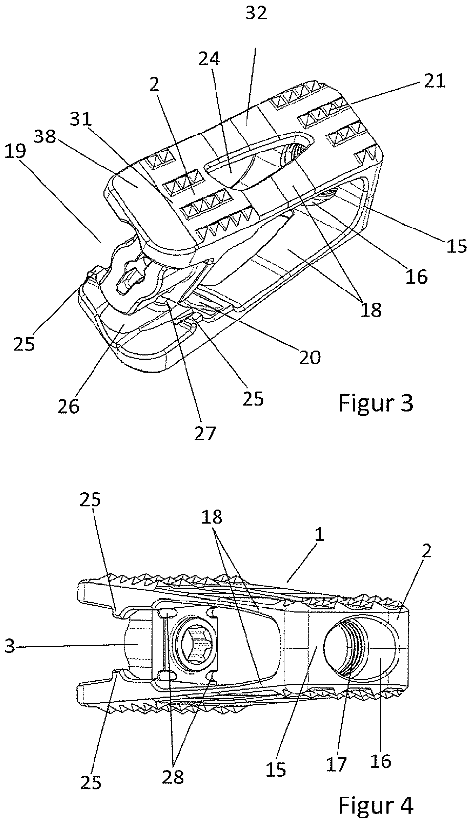

Figur 1 zeigt einen horizontalen medialen Längsschnitt durch den monolithischen Hauptkörper (2) eines erfindungsgemäßen Cages (1) mit auf dem unteren Arm (18) in expandierter Lage eingelegtem Spreizelement (3). Das Spreizelement (3) weist einen drehbaren, länglichen, zylindrischen Grundkörper (4) auf, an dessen hinterem Ende eine rechteckige Platte (5) mit in Längsrichtung des Cages (1) verlaufenden, abgerundeten Kanten (6) einstückig angeformt ist. Von der Platte (5) bis zum vorderen Ende des Grundkörpers (4) des Spreizelementes (3) sind zwei einstückige, gegenüberliegende Rippen (8) ausgebildet, deren jeweilige radiale Auflageflächen (9) mit den kürzeren Schmalseiten (33) der rechteckigen Platte (5) abschließen. Die Breite der Rippen (8) erreicht nahezu den Durchmesser des zylindrischen Grundkörpers (4). Jeweils rechteckige Nuten (10) sind in die Rippen (8) so eingebracht, dass sie parallel zur kürzeren Schmalseite (33) der rechteckigen Platte (5) verlaufen und eine Seitenfläche mit der vorderen Deckfläche der Platte (5) zusammen fällt. In Spreizstellung des Spreizelementes (3) lagern die jeweils paarweisen gegenüberliegenden gekrümmten Auflageflächen (9) in Rinnen (26), die in Längsrichtung des Cages (1) verlaufen und an den Innenseiten der Arme (18) vorhanden sind. Das Spreizelement (3) weist im hinteren Teil des Grundkörpers (4) einen von posterior zugänglichen Innensechsrund (12) (Figur 4) auf, der zum Drehen des Spreizelementes (3) dient.

Das Spreizelement (3) besitzt, wie Figur 2 zeigt, eine mittige Bohrung (11). In die vordere Oberfläche des Spreizelements (3) sind zwei Nuten (14) zur besseren Revision eingebracht, welche in Richtung der Rippen (8) verlaufen, ein Stück vor der Auflagefläche (9) enden und deren Breite geringer wie der Durchmesser der Bohrung (11) ist. Die Nuten sind nicht durch den Körper durchgehend, weisen also eine eingeschränkte Tiefe auf.

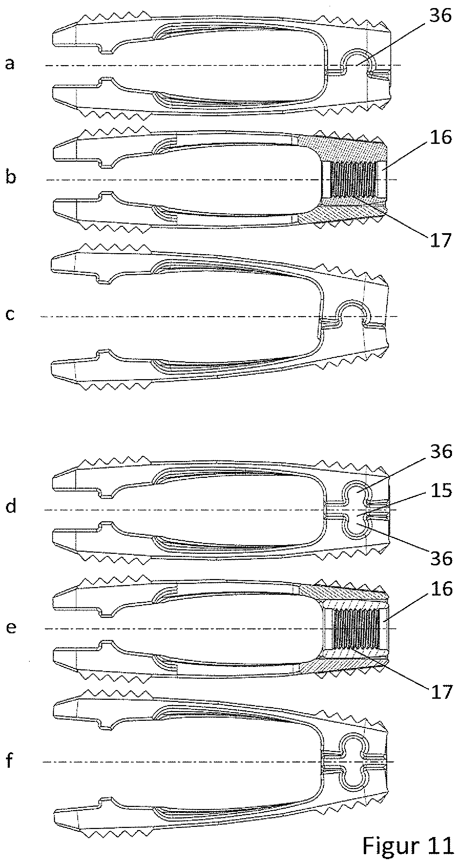

Wie Figur 3 erkennen lässt und insbesondere aus Figur 4 ersichtlich ist, weist der Hauptkörper (2) des Cages (1) im medialen Längsschnitt ein im wesentlichen U-förmiges Profil auf, wobei sein anteriores Ende offen ist. Zwei annähernd parallel zueinander verlaufende spreizbare Arme (18), die jeweils als Stützflächen (32) für benachbarte Wirbelkörper dienen, sind über eine brückenähnliche Verbindung (15) miteinander verbunden. Im dargestellten Beispiel ist die brückenähnliche Verbindung (15) eine direkte, einstückig mit den Armen (18) ausgebildete Verbindung, so dass ein monolithischer Hauptkörper (2) vorliegt. Als Alternativen können die beiden Arme (18) aber auch über brückenähnliche Verbindungen mit einem oder zwei scharnierartigen Gelenken (36) miteinander verbunden sein, wie in Figur 11 gezeigt.

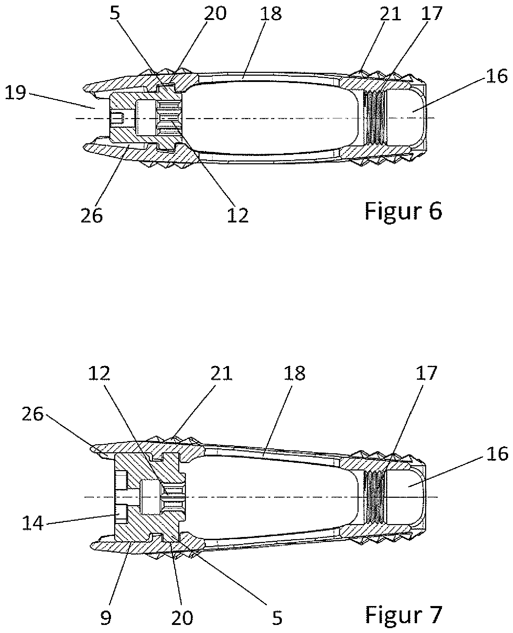

Die Verbindung (15) weist eine zentrale Öffnung (16) zum Einführen von Instrumenten auf. Diese Öffnung (16) ist am hinteren Ende zylindrisch ausgebildet, um anschließend in ein Schraubengewinde (17, Figur 4) überzugehen. Die Stärke der Arme (18) nimmt zur Mitte hin ab, sodass im medialen Längsschnitt eine ellipsenartige Öffnung entsteht. Am vorderen Ende sind die Arme (18) soweit verstärkt, dass ein durch sie gebildeter Spalt (19) ein Öffnungsmaß aufweist, welches der Breite der Rippen (8) entspricht, wenn der Cage (1) nicht gespreizt ist (Figur 6). In die verstärkten Enden der Arme (18) sind vom Spalt (19) aus gesehen gegenüberliegende Nuten (20) vorhanden, in welche die Platte (5) so eingreift, dass das Spreizelement (3) in Längsrichtung des Cages (1) fixiert ist. Auch sind in die verstärkten Enden der Arme (18) mittig zylinderförmige Rinnen (26) eingearbeitet, deren Radius gleich dem Radius der Auflageflächen (9) der Rippen (8) des Grundkörpers (4) ist. Die Länge der Rinnen (26) im medialen Längsschnitt ist so bemessen, dass ein Steg (27) derart verbleibt, dass dieser in die Nut (10) des Spreizelementes (3) eingreift und mit Platte (5) in Kontakt ist.

Im nicht expandierten Zustand des Cages (1) liegen die Rippen (8) transversal und der Hauptkörper (2) ist ungespreizt. Die zylindrische Mantelfläche des Grundkörpers (4) liegt vollflächig in den Rinnen (26) der verdickten Enden der Arme (18). Die als Stützflächen (32) ausgebildeten Außenseiten (31) der Arme (18) sind mit einer Profilierung (21) versehen (Fig. 3).

Figur 4 zeigt den Cage (1) mit Hauptkörper (2) und Spreizelement (3) von der posterioren Seite aus gesehen im expandierten Zustand. Deutlich erkennbar sind seitliche Ausfräsungen (28) im Spreizelement (3), welche für eine radiologische Lagekontrolle genutzt werden können. Auch sind die Öffnung (16) und das Schraubengewinde (17) dargestellt.

Da die Verbindung (15) einstückig in die Arme (18) übergeht, ermöglicht diese Ausgestaltung eine größtmögliche laterale Aussparung durch die offenen, in Längsrichtung verlaufenden offenen Seiten des Cages (1), da dies die beste radiologische Durchdringungsmöglichkeit sicherstellt. Notwendig ist eine Verformbarkeit der Arme (18) beim Spreizen. Die Verformbarkeit wird weiterhin durch die geometrische Ausführung der Arme (18) begünstigt.

Seitlich der verstärkten Enden der Arme (18) sind kleine Haken (25) ausgebildet, welche ein Herausspringen des Spreizelementes (3) in eine Fehlstellung verhindern.

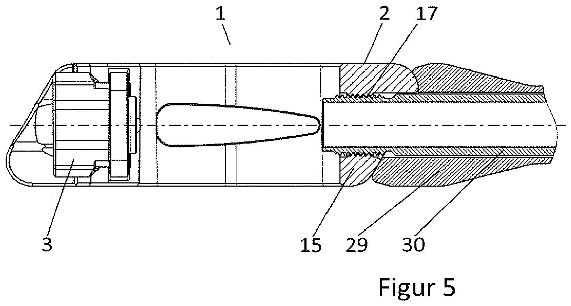

Die Anbindung eines Instrumentes (29) an den Cage (1) bzw. die Verbindung (15) zeigt Figur 5 und ist so gestaltet, dass keine zusätzliche Aussparung im Cage (1) notwendig ist. Dazu wird ein Rohr (30) in das Schraubengewinde (17) eingeschraubt um das Instrument (29) mit dem Cage (1) verdrehsicher zu verbinden, wobei die schräg verlaufende Verbindung (15) für die Sicherung gegen Verdrehen von Vorteil ist. Auch kann durch das Rohr (30) vorzugsweise synthetisches Knochenmaterial in den Cage (1) eingeführt werden.

Figur 6 zeigt den nicht expandierten Cage (1) im medialen vertikalen Längsschnitt, wie er zwischen die Wirbelkörper eingebracht wird. Die Auflageflächen (9) des Grundkörpers (4) liegen in den jeweiligen Rinnen (26). In die Nut (20) greift die Platte (5) mit ihrer längeren Schmalseite (33) ein. Mittels eines geeigneten, nicht dargestellten Werkzeugs mit einem Sechsrund an seiner Spitze, der in den Innensechsrund (12) einführbar ist, muss, um den Cage (1) in den expandierten Zustand zu bringen, das Spreizelement (3) nur um 90° gedreht werden. Die Ausführungsform ermöglicht beide Drehrichtungen, so dass sowohl durch ein Zurückdrehen um 90° als auch durch ein Weiterdrehen um 90° eine Zurücknahme der Expansion erfolgt.

Figur 7 zeigt den expandierten Cage (1) im medialen vertikalen Längsschnitt. Die gekrümmten Auflageflächen (9) der Rippen haben in dieser Stellung flächigen Kontakt mit den Rinnen (26) und gleichzeitig liegt die Platte (5) mit ihren kürzeren Schmalseiten (33) in den Nuten (20) flächig auf. Damit ist eine sichere und kippsichere Lastabtragung mit größtmöglicher Auflagefläche in T-Form (vgl. Figur 1) sichergestellt. Auch zeigt Figur 7 noch die Nuten (14), in welche ein geeignetes Werkzeug eingreifen kann, um das Spreizelement (3) nötigenfalls bei einer Revisions-OP von anterior zu entfernen. Ebenso ist eine hintere Vorrichtung (12) zum drehbaren Antrieb des Spreizelementes (3) dargestellt.

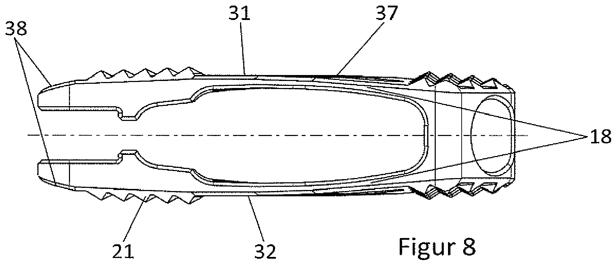

Figur 8 zeigt den erfindungsgemäßen Hauptkörper (2) des Cages (1) nach Figur 4 in seitlicher, nicht gespreizter Ansicht. Die die Stützflächen (32) bildenden Außenseiten (31) der Arme (18) sind in Längsrichtung des Cages (1) leicht gewölbt. Auch ist jeweils eine Einführfase (38) am vorderen Ende der Arme (18) sichtbar. Zähne (21) sind im vorderen und im hinteren Bereich der Stützflächen (32) zur Primärverankerung lokalisiert.

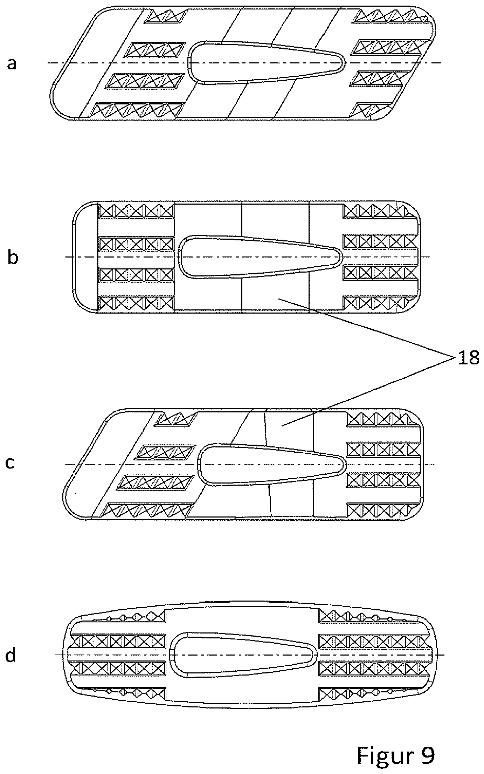

Figuren 9 a - d zeigen verschiedene Grundformen erfindungsgemäßer Cages (1) in Draufsicht. Figur 9 a zeigt beispielsweise eine parallelogrammförmige Grundform. Sie gewährleistet bei transforaminaler Operationstechnik bei schräger Einbringung und Lage eine bessere Lastabtragung, da der Cage (1) annähernd mit dem Wirbelkörperrandbereich abschließt. Figur 9 b zeigt eine mögliche rechteckige Grundform. Figur 9 c zeigt, dass die Grundform auch viereckig mit unterschiedlich langen Seiten sein kann, wobei zwei rechte Winkel an einer kurzen Seite vorhanden sind. Figur 9 d zeigt eine überwiegend elliptische Grundform der Arme (18), die insbesondere beim Einführen des Cages einen gering invasiven Eingriff erleichtern. Auch sind die verschiedenen Möglichkeiten dargestellt, wie die Zähne (21) auf den Armen (18) angeordnet sein können.

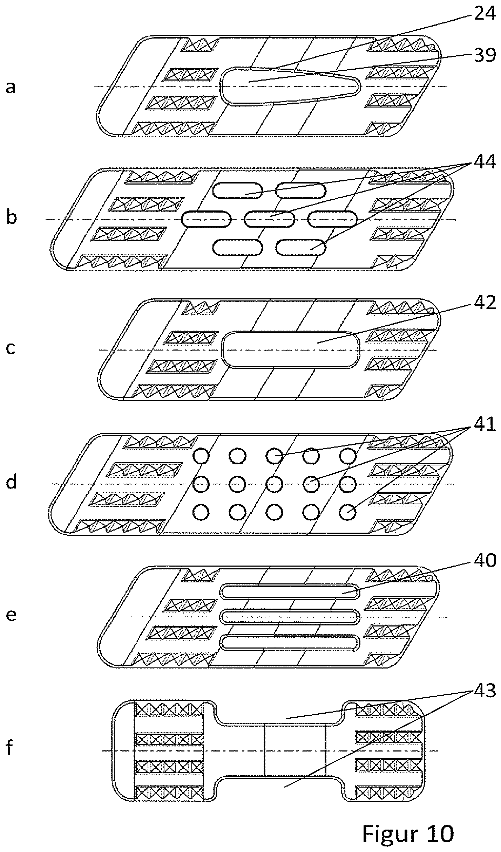

Figuren 10 a - f zeigen verschieden Formen vertikaler Aussparungen (24) in den Armen (18) zur Durchbauung mit Knochengewebe als sekundäre Verankerung. In Figur 10 a ist eine Aussparung (24) als ein im Wesentlichen teilelliptisch nach posterior kleiner werdendes Fenster (39) ausgestaltet. In Figur 10 b sind Aussparungen (24) in Form von Langlöchern (44) vorhanden, die in parallel zueinander verlaufenden Reihen mit zwei oder drei Langlöchern pro Reihe verlaufen. Die vertikalen Aussparungen (24) können auch als im Wesentlichen rechteckiges Fenster (42) ausgestaltet sein, wie Figur 10 c zeigt. Figur 10 d zeigt eine Form der Aussparungen (24), die aus hintereinander liegenden Bohrungen (41) besteht, die in Längsrichtung des Cages (1) in mehreren parallel zueinander verlaufenden Reihen matrixähnlich angeordnet sind. Die Aussparung (24) kann auch aus drei schmalen, gleich langen in Längsrichtung des Cages (1) parallel zueinander verlaufenden Durchbrechungen (40) bestehen, wie sie in Figur 10 e dargestellt sind. Figur 10 f zeigt vertikale Aussparungen (24), die aus paarweisen seitlichen Ausbrüchen (43) an den Längsseiten der Arme (18) bestehen.

Figuren 11 a - c und 11 d - f zeigen Cages (1) mit einem bzw. zwei scharnierartigen Gelenken (36), die jeweils aus zwei seitlich zur Öffnung (16) angebrachten drehbaren Verbindungen bestehen. Figur 11 a zeigt einen Cage (1) mit einem scharnierartigen Gelenk (36) im nicht gespreizten Zustand. Figur 11 b zeigt diesen Cage (1) im Teilschnitt der Verbindung (15), die zwischen den drehbaren Verbindungen des scharnierartigen Gelenks (36) vorhanden ist. Die Öffnung (16) und das Schraubengewinde (17) zum Konnektieren von Instrumenten an den Cage (1) ist deutlich zu erkennen. Figur 11 c zeigt diesen Cage (1) im gespreizten Zustand. Die Verbindung (15) ist dabei vorteilhaft in das Gelenk (36) umgestaltet. Figur 11d zeigt einen Cage (1) mit zwei scharnierartigen Gelenken (36) im nicht gespreizten Zustand. Figur 11e zeigt diesen Cage (1) im Teilschnitt der Verbindung (15), die zwischen den drehbaren Verbindungen der Gelenke (36) vorhanden ist. Die Öffnung (16) und das Schraubengewinde (17) sind ebenfalls klar erkennbar. Figur 11 f zeigt diesen Cage (1) im gespreizten Zustand. Die Verbindung (15) bildet hier in vorteilhafter Weise die gemeinsame Basis für die vertikal übereinanderliegenden drehbaren Verbindungen der beiden Gelenke (36). Beide Varianten weisen gegenüber einer monolithischen Ausgestaltung des Hauptkörpers den Vorteil auf, dass die Arme (18) bei der Expansion weniger stark verformt werden.

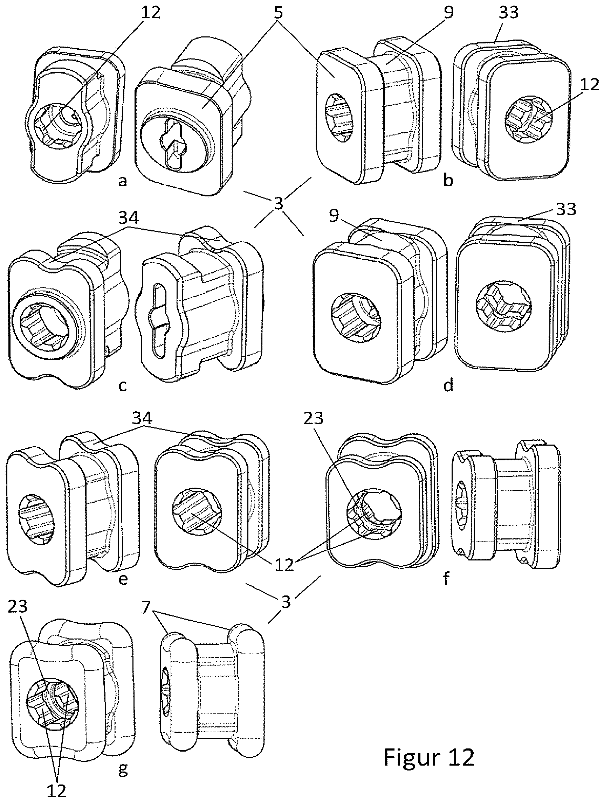

Figuren 12 a - g zeigen verschiedene im Rahmen der Erfindung liegende Spreizelemente (3), die sowohl im hinteren als auch im vorderen Teil Vorrichtungen zum drehbaren Antrieb aufweisen.

Figur 12 a zeigt ein Spreizelement (3) wie beispielsweise in der Darstellung in Figur 1 gezeigt, wobei hier aber die Vorrichtungen zum drehbaren Antrieb des Cages (1) vertauscht sind.

Das Spreizelement (3) in Figur 12 b zeichnet sich durch zwei rechteckige Platten (5) an beiden Enden des länglichen Grundkörpers (4) aus, die gleich dimensioniert sind. Die Auflageflächen (9) schließen nicht mit den Oberflächen der kürzeren Schmalseiten (33) ab. In jede Platte (5) ist ein Innensechsrund (12) eingearbeitet.

Das Spreizelement (3) nach Figur 12 c zeichnet sich durch sickenförmige Ausnehmungen (34) in den kürzeren Schmalseiten (33) der rechteckigen Platte (5) zur verbesserten Arretierung im expandierten Zustand des Cages aus.

Figur 12 d zeigt ein Spreizelement (3) mit zwei gleich dimensionierten Platten (5), wobei die Auflagefläche (9) mit den Oberflächen der kürzeren Schmalseiten (33) abschießt, so dass sich eine H-förmige, in einer Ebene liegende flächige Auflage ergibt.

Das Spreizelement (3) nach Figur 12 e unterscheidet sich vom Spreizelement gemäß Figur 12 b dadurch, dass zusätzliche, der verbesserten Arretierung dienende sickenförmige Ausnehmungen (34) in beiden kürzeren Schmalseiten (33) der Platten (5) eingearbeitet sind, deren Tiefe bis zur Höhe der Auflageflächen (9) reicht.

Figur 12 f zeigt ein ähnliches Spreizelement (3) wie nach Figur 12 e mit dem Unterschied, dass eine der Platten (5) die andere in Höhe und Breite überragt.

Figur 12 g zeigt ein Spreizelement (3) bei dem alle äußeren Ecken und Kanten des Spreizelementes (3) gebrochen oder mit einem Radius versehen sind.

Die Figuren 12 f und 12 g zeigen in den Spreizelementen (3) sowohl im hinteren als auch im vorderen Teil Innensechsrunde (12), zwischen denen jeweils ein Bund (23) erkennbar ist, der als Innendurchbruchsicherung dient.

Die Figur 12 e zeigt ein Spreizelement (3), bei welchem der Innensechsrund (12) vom vorderen bis zum hinteren Teil durchgehend ausgeführt ist.

Alle dargestellten Ausführungsformen stellen in vorteilhafter Weise zusätzlich sicher, dass die entsprechenden Cages (1) im expandierten Zustand kippsicher und zuverlässig arretiert sind, wobei selbstverständlich die verdickten Enden der Arme (18) jeweils an ein Spreizelement (3) entsprechend den Figuren 12 a - g angepasst sind. Mit den kreuzweise angeordneten Nuten (20) und Rinnen (26) wird jeweils eine sichere Lagefixierung erzielt.

### Bezugszeichen liste

- 1: Cage
- 2: Hauptkörper
- 3: Spreizelement
- 4: Grundkörper
- 5: Platten
- 6: Kanten
- 7: Radius
- 8: Rippen
- 9: Auflageflächen
- 10: Nuten
- 11: Bohrung
- 12: Innensechsrund
- 13: (frei)
- 14: Nuten
- 15: Verbindung
- 16: Öffnung
- 17: Schraubengewinde
- 18: Arme
- 19: Spalt
- 20: Nuten
- 21: Zähne
- 22: Stützfläche
- 23: Bund
- 24: Aussparung
- 25: Haken
- 26: Rinne
- 27: Steg
- 28: Ausfräsungen
- 29: Instrument
- 30: Rohr
- 31: Außenseiten der Arme
- 32: Stützfläche
- 33: Schmalseiten der Platten
- 34: sickenförmige Ausnehmungen
- 36: scharnierartige Gelenke
- 37: Wölbung
- 38: Einführfase
- 39: teilelliptisches Fenster
- 40: Durchbrechung
- 41: Bohrungen
- 42: rechteckiges Fenster
- 43: seitliche Ausbrüche
- 44: Langlöcher

## Patentansprüche

1. Expandierbarer Cage (1) zur interkorporellen Fusion von Lendenwirbeln
- mit zwei im Wesentlichen parallel zueinander verlaufenden spreizbaren Armen (18), deren Außenseiten (31) jeweils als Stützflächen (32) für benachbarte Wirbelkörper ausgebildet sind,
- mit einer an einem Ende der Arme (18) bestehenden brückenähnlichen Verbindung (15), so dass sich im medialen Längsschnitt ein im wesentlichen U-förmiges Profil des Hauptkörpers (2) des Cages (1) ergibt,
- mit einem am anderen, freien Ende der Arme (18) befindlichen und zwischen diesen drehbar gelagerten Spreizelement (3) zum Auseinanderdrücken der Arme (18) und Expandieren des Cages (1) und
- mit einer in der brückenähnlichen Verbindung (15) vorgesehenen Öffnung (16) zum Konnektieren von Instrumenten an den Cage (1),
**dadurch gekennzeichnet**
- **dass** das Spreizelement (3) aus einem drehbaren länglichen, insbesondere zylinderförmigen Grundkörper (4) besteht, der ein oder zwei einstückig damit verbundene rechteckige Platten (5) mit in Längsrichtung des Cages (1) verlaufenden abgerundeten Kanten (6) sowie zwei gegenüberliegende, radial ausgerichtete und in Längsrichtung des Cages (1) verlaufende Rippen (8) aufweist,
- **dass** die rechteckigen Platten (5) mit ihren Schmalseiten (33) in korrespondierende Nuten (20), die in den Innenseiten der Arme (18) quer verlaufen, sowohl im nicht expandierten als auch im expandierten Zustand des Cages (1) eingreifen,
- **dass** sowohl die Rippen (8) als auch der Grundkörper (4) jeweils paarweise gegenüberliegende gekrümmte Auflageflächen (9) an ihrem jeweiligen äußeren Umfang aufweisen, die alternativ und abhängig vom Spreizzustand des Cages (1) in korrespondierenden, in Längsrichtung des Cages (1) verlaufenden und an den Innenseiten der Arme (18) vorhandenen Rinnen (26) zur drehbaren Lagerung aufliegen, und
- **dass** zumindest im hinteren, zur brückenähnlichen Verbindung (15) hin liegenden Teil des Spreizelementes (3) eine Vorrichtung zum drehbaren Antrieb des Spreizelementes (3) vorhanden ist.

2. Expandierbarer Cage (1) zur interkorporellen Fusion von Lendenwirbeln nach dem vorliegenden Anspruch 1, **dadurch gekennzeichnet,**
- **dass** die im hinteren Teil des Spreizelementes (3) vorhandene Vorrichtung zum drehbaren Antrieb des Spreizelementes als Vielzahn-Verbindung, vorzugsweise als Innensechsrund (12) ausgeführt ist.

3. Expandierbarer Cage (1) zur interkorporellen Fusion von Lendenwirbeln nach dem vorliegenden Anspruch 2, **dadurch gekennzeichnet,**
- **dass** im vorderen Teil des Spreizelementes (3) eine weitere Vorrichtung zum drehbaren Antrieb des Spreizelementes (3) positioniert ist, die aus einer mittigen, durch den Grundkörper hindurch gehenden Bohrung (11) mit zwei davon abgehenden und in Richtung der Rippen (8) verlaufenden, aber nicht bis zu deren jeweilige Auflageflächen (9) sich erstreckenden und eine eingeschränkte Tiefe aufweisenden Nuten (14) besteht, deren Breite geringer als der Durchmesser der Bohrung (11) ist.

4. Expandierbarer Cage (1) zur interkorporellen Fusion von Lendenwirbeln nach einem der Ansprüche 1 bis 3, **dadurch gekennzeichnet,**
- **dass** die Bodenflächen der als trapezförmige Innentaschen ausgeformten Nuten (20) in den Innenseiten der Arme (18) im expandierten Zustand des Cages (1) flächig auf den kürzeren Schmalseiten (33) der rechteckigen Platten (5) aufliegen.

5. Expandierbarer Cage (1) zur interkorporellen Fusion von Lendenwirbeln nach einem der Ansprüche 1 bis 4, **dadurch gekennzeichnet,**
- **dass** in den kürzeren Schmalseiten (33) der rechteckigen Platten (5) mittig sickenförmige Ausnehmungen (34) und in den Bodenflächen der in den Innenflächen der Arme (18) ausgeformten Nuten (20) mittig korrespondierende Erhebungen ausgeformt sind.

6. Expandierbarer Cage (1) zur interkorporellen Fusion von Lendenwirbeln nach einem der Ansprüche 1 bis 5, **dadurch gekennzeichnet,**
- **dass** am Spreizelement (3) seitliche Ausfräsungen (28) in mindestens einer der Platten (5) vorhanden sind, die eine genaue Lagekontrolle des Spreizelementes (3) im Cage (1) über ein bildgebendes Verfahren erlauben.

7. Expandierbarer Cage (1) zur interkorporellen Fusion von Lendenwirbeln nach einem der Ansprüche 1 bis 6, **dadurch gekennzeichnet,**
- **dass** auf den Innenseiten der Arme (18) kleine seitliche Haken (25) vorgesehen sind, die ein Herausspringen des Spreizelementes in eine Fehlstellung verhindern.

8. Expandierbarer Cage (1) zur interkorporellen Fusion von Lendenwirbeln nach einem der Ansprüche 1 bis 7, **dadurch gekennzeichnet,**
- **dass** die in der brückenähnlichen Verbindung (15) befindliche Öffnung (16) als zylinderförmiger Bohrung (35) mit einem Ansatz für ein Schraubengewinde (17) ausgestaltet ist.

9. Expandierbarer Cage (1) zur interkorporellen Fusion von Lendenwirbeln nach einem der Ansprüche 1 bis 8, **dadurch gekennzeichnet,**
- **dass** die brückenähnliche Verbindung (15) zwischen den Armen (18) einstückig mit diesen ausgebildet ist, so dass ein monolithischer Hauptkörper (2) gegeben ist.

10. Expandierbarer Cage (1) zur interkorporellen Fusion von Lendenwirbeln nach einem der Ansprüche 1 bis 8, **dadurch gekennzeichnet,**
- **dass** die brückenähnliche Verbindung (15) zwischen den Armen (18) mindestens ein scharnierartiges Gelenk (36) aufweist.

11. Expandierbarer Cage (1) zur interkorporellen Fusion von Lendenwirbeln nach einem der Ansprüche 1 bis 10, **dadurch gekennzeichnet,**
- **dass** die als Stützflächen (32) für benachbarte Wirbelkörper ausgebildeten Außenseiten der Arme (31) eine leichte Wölbung (37) in Längsrichtung des Cages (1) aufweisen, am vorderen Ende eine Einführfase (38) besitzen und an den Stützflächen für die benachbarten Wirbelkörper Zähne (21) zur Primärverankerung aufweisen.

12. Expandierbarer Cage (1) zur interkorporellen Fusion von Lendenwirbeln nach einem der Ansprüche 1 bis 11, **dadurch gekennzeichnet,**
- **dass** die Grundform der Arme (18) in Draufsicht einem Viereck mit unterschiedlich langen Seiten und zwei rechten Winkeln entspricht.

13. Expandierbarer Cage (1) zur interkorporellen Fusion von Lendenwirbeln nach einem der vorangegangenen Ansprüche 1 bis 12, **dadurch gekennzeichnet,**
- **dass** alle äußeren Ecken und Kanten des Spreizelementes (3) gebrochen oder mit einem Radius (7) versehen sind.

## Claims

1. Expandable cage (1) for intercorporal fusion of lumbar vertebrae
- with two expendable arms (18) running substantially parallel to each other, whose outsides (31) are each constructed as supporting surfaces (32) for adjacent vertebral bodies,
- with a bridge-like connection (15) present at one end of the arms (18), so that in the medial longitudinal section a substantially U-shaped profile of the main body (2) of the cage (1) is obtained,
- with an expansion element (3), being located at the other, free end of the arms (18) and being rotatably mounted between these, for pressing the arms (18) apart and expanding the cage (1), and
- with an opening (16) provided in the bridge-like connection (15) for connecting instruments to the cage (1),
**characterized in**
- **that** the expansion element (3) comprises a rotatable elongated, in particular cylindrical, base body (4) which has one or two rectangular plates (5) which are integrally connected thereto and which have rounded edges (6) running in the longitudinal direction of the cage (1) and two opposing, radially aligned ribs (8) running in the longitudinal direction of the cage (1),
- **that** the rectangular plates (5) engage with their narrow sides (33) in corresponding grooves (20), which extend transversely in the insides of the arms (18), both in the unexpanded and expanded state of the cage (1),
- **that** both the ribs (8) and the base body (4) each have curved, pair-wise opposing bearing surfaces (9) along their respective outer circumference, which, alternatively, and depending on the expansion state of the cage (1), lie for rotatable support in corresponding channels (26) which extend in the longitudinal direction of the cage (1) and are present on the insides of the arms (18), and
- **that** a device for rotatably driving the expansion element (3) is present at least in the rear part of the expansion element (3) located towards the bridge-like connection (15).

2. Expandable cage (1) for intercorporal fusion of lumbar vertebrae according to the present Claim 1, **characterized in**
- **that** the device present in the rear part of the expansion element (3) for rotatably driving the expansion element is constructed as a multi-tooth connection, preferably as a hexalobular socket (12).

3. Expandable cage (1) for intercorporal fusion of lumbar vertebrae according to the present Claim 2, **characterized in**
- **that** positioned in the front part of the expansion element (3), there is a further device for rotatably driving the expansion element (3), which comprises a central bore (11) passing through the base body and having two grooves (14), which project from the bore and continue in the direction of the ribs (8) but do not extend so far as their respective bearing surfaces (9), and have a restricted depth and a width which is smaller than the diameter of the bore (11).

4. Expandable cage (1) for intercorporal fusion of lumbar vertebrae according to one of Claims 1 to 3, **characterized in**
- **that** the base surfaces of the grooves (20) formed as trapezoidal inside pockets in the insides of the arms (18) lie flat against the shorter narrow sides (33) of the rectangular plates (5) in the expanded state of the cage (1).

5. Expandable cage (1) for intercorporal fusion of lumbar vertebrae according to one of Claims 1 to 4, **characterized in**
- **that** bead-shaped recesses (34) are formed centrally in the shorter narrow sides (33) of the rectangular plates (5) and corresponding raised portions are formed centrally in the base surfaces of the grooves (20) formed in the inner surfaces of the arms (18).

6. Expandable cage (1) for intercorporal fusion of lumbar vertebrae according to one of Claims 1 to 5, **characterized in**
- **that** on the expansion element (3), lateral notches (28) are present in at least one of the plates (5), which enable precise position control of the expansion element (3) in the cage (1) using an imaging technique.

7. Expandable cage (1) for intercorporal fusion of lumbar vertebrae according to one of Claims 1 to 6, **characterized in**
- **that** small lateral hooks (25) which prevent the expansion element from springing out into a faulty position are provided on the insides of the arms (18).

8. Expandable cage (1) for intercorporal fusion of lumbar vertebrae according to one of Claims 1 to 7, **characterized in**
- **that** the opening (16) located in the bridge-like connection (15) is constructed as a cylindrical bore (35) with a projection for a screw thread (17).

9. Expandable cage (1) for intercorporal fusion of lumbar vertebrae according to one of Claims 1 to 8, **characterized in**
- **that** the bridge-like connection (15) between the arms (18) is integrally formed therewith so that a monolithic main body (2) is given.

10. Expandable cage (1) for intercorporal fusion of lumbar vertebrae according to one of Claims 1 to 8, **characterized in**
- **that** the bridge-like connection (15) has at least one hinge-like joint (36) between the arms (18).

11. Expandable cage (1) for intercorporal fusion of lumbar vertebrae according to one of Claims 1 to 10, **characterized in**
- **that** the outsides of the arms (31), which are constructed as supporting surfaces (32) for adjacent vertebral bodies, have a slight curvature (37) in the longitudinal direction of the cage (1), possess a lead-in chamfer (38) at the front end and have teeth (21) on the supporting surfaces for the adjacent vertebral bodies for the purpose of primary anchoring.

12. Expandable cage (1) for intercorporal fusion of lumbar vertebrae according to one of Claims 1 to 11, **characterized in**
- **that** the basic shape of the arms (18) in plan-view corresponds to a quadrilateral with sides of different lengths and two right-angles.

13. Expandable cage (1) for intercorporal fusion of lumbar vertebrae according to one of the preceding Claims 1 to 12, **characterized in**
- **that** all the outer corners and edges of the expansion element (3) are blunted or provided with a radius (7).

## Revendications

1. Cage expansible (1) pour la fusion intracorporelle de vertèbres lombaires,
- avec deux bras expansibles (18) s'étendant sensiblement parallèlement entre eux, dont les côtés extérieurs (31) sont respectivement réalisés en tant que surfaces de soutien (32) pour des corps vertébraux voisins,
- avec une liaison du genre pont (15) existante à une extrémité des bras (18), de sorte qu'on obtient, en coupe longitudinale médiale, un profil sensiblement en forme de U du corps principal (2) de la cage (1),
- avec un élément d'expansion (3), se trouvant à l'autre extrémité, libre, des bras (18) et monté à rotation entre ceux-ci, pour l'écartement des bras (18) l'un de l'autre et l'expansion de la cage (1),
- et avec une ouverture (16) prévue dans la liaison du genre pont (15), pour connecter des instruments à la cage (1),
**caractérisée**
- **en ce que** l'élément d'expansion (3) est constitué d'un corps de base oblong rotatif (4), en particulier cylindrique, qui présente une ou deux plaques rectangulaires (5) reliées à lui d'un seul tenant et ayant des bords arrondis (6) s'étendant dans la direction longitudinale de la cage (1), ainsi que deux nervures opposées (8), orientées radialement et s'étendant dans la direction longitudinale de la cage (1),
- **en ce que** les plaques rectangulaires (5), tant dans l'état non expansé que dans l'état expansé de la cage (1), s'engagent par leurs côtés étroits (33) dans des rainures correspondantes (20) qui s'étendent transversalement dans les côtés intérieurs des bras (18),
- **en ce que** tant les nervures (8) que le corps de base (4) présentent sur leur pourtour extérieur respectif des surfaces d'appui incurvées (9) respectivement opposées par paires qui, pour le montage à rotation, reposent alternativement et en fonction de l'état d'expansion de la cage (1) dans des conduits correspondants (26) s'étendant dans la direction longitudinale de la cage (1) et présents sur les côtés intérieurs des bras (18),
- et **en ce qu'**un dispositif pour l'entraînement en rotation de l'élément d'expansion (3) est présent au moins dans la partie arrière de l'élément d'expansion (3), située vers la liaison du genre pont (15).

2. Cage expansible (1) pour la fusion intracorporelle de vertèbres lombaires selon la revendication précédente 1, **caractérisée en ce que** le dispositif présent dans la partie arrière de l'élément d'expansion (3) pour l'entraînement en rotation de l'élément d'expansion est réalisé sous forme de liaison polygonale, de préférence sous forme de tête ronde à empreinte étoile 6 branches (12).

3. Cage expansible (1) pour la fusion intracorporelle de vertèbres lombaires selon la revendication précédente 2, **caractérisée en ce qu'**un autre dispositif pour l'entraînement en rotation de l'élément d'expansion (3) est positionné dans la partie avant de l'élément d'expansion (3), dispositif qui est constitué d'un perçage central (11), s'étendant à travers le corps de base et pourvu de deux rainures (14) partant de ce perçage et s'étendant en direction des nervures (8), mais non jusqu'à leurs surfaces d'appui respectives (9), rainures qui présentent une profondeur limitée et dont la largeur est inférieure au diamètre du perçage (11).

4. Cage expansible (1) pour la fusion intracorporelle de vertèbres lombaires selon l'une des revendications 1 à 3, **caractérisée en ce que** les surfaces de base des rainures (20) façonnées dans les côtés intérieurs des bras (18) sous la forme de poches intérieures trapézoïdales reposent, dans l'état expansé de la cage (1), à plat sur les côtés étroits plus courts (33) des plaques rectangulaires (5).

5. Cage expansible (1) pour la fusion intracorporelle de vertèbres lombaires selon l'une des revendications 1 à 4, **caractérisée en ce que** des évidements (34) en forme de moulures sont façonnés centralement dans les côtés étroits plus courts (33) des plaques rectangulaires (5) et des bossages correspondants sont façonnés centralement dans les surfaces de base des rainures (20) façonnées dans les faces intérieures des bras (18).

6. Cage expansible (1) pour la fusion intracorporelle de vertèbres lombaires selon l'une des revendications 1 à 5, **caractérisée en ce que** des fraisures latérales (28) sont présentes sur l'élément d'expansion (3) dans au moins une des plaques (5), fraisures qui permettent un contrôle précis de la position de l'élément d'expansion (3) dans la cage (1) au moyen d'un procédé d'imagerie.

7. Cage expansible (1) pour la fusion intracorporelle de vertèbres lombaires selon l'une des revendications 1 à 6, **caractérisée en ce que** des petits crochets latéraux (25) sont prévus sur les côtés intérieurs des bras (18), crochets qui empêchent l'élément d'expansion de sauter dans une mauvaise position.

8. Cage expansible (1) pour la fusion intracorporelle de vertèbres lombaires selon l'une des revendications 1 à 7, **caractérisée en ce que** l'ouverture (16) se trouvant dans la liaison du genre pont (15) est réalisée sous forme de perçage cylindrique (35) pourvu d'un embout pour un filetage de vis (17).

9. Cage expansible (1) pour la fusion intracorporelle de vertèbres lombaires selon l'une des revendications 1 à 8, **caractérisée en ce que** la liaison du genre pont (15) entre les bras (18) est réalisée d'un seul tenant avec ceux-ci, de sorte qu'on obtient un corps principal monolithique (2).

10. Cage expansible (1) pour la fusion intracorporelle de vertèbres lombaires selon l'une des revendications 1 à 8, **caractérisée en ce que** la liaison du genre pont (15) entre les bras (18) présente au moins une articulation (36) du genre charnière.

11. Cage expansible (1) pour la fusion intracorporelle de vertèbres lombaires selon l'une des revendications 1 à 10, **caractérisée en ce que** les côtés extérieurs (31) des bras (18), conçus comme surfaces de soutien (32) pour des corps vertébraux voisins, présentent une légère cambrure (37) dans la direction longitudinale de la cage (1), possèdent un chanfrein d'introduction (38) à l'extrémité avant et présentent, sur les surfaces de soutien pour les corps vertébraux voisins, des dents (21) pour l'ancrage primaire.

12. Cage expansible (1) pour la fusion intracorporelle de vertèbres lombaires selon l'une des revendications 1 à 11, **caractérisée en ce que** la forme de base des bras (18) correspond, en vue de dessus, à un quadrilatère avec des côtés de longueurs différentes et deux angles droits.

13. Cage expansible (1) pour la fusion intracorporelle de vertèbres lombaires selon l'une des revendications 1 à 12, **caractérisée en ce que** tous les bords et les coins extérieurs de l'élément d'expansion (3) sont brisés ou dotés d'un rayon (7).
